# EUROPÄISCHE PATENTANMELDUNG

(11) **EP 2 940 130 A1**
(43) Veröffentlichungstag der Anmeldung: **04.11.2015**
(21) Anmeldenummer: 14166356.7
(22) Anmeldetag: 29.04.2014
(51) Int. Cl.: C12N 11/00, C12N 11/02, C12N 11/06, C12N 11/08, C12N 11/10, C12N 11/12

(54) **Mit Metalloxidpartikeln oberflächenmodifizierte Träger und immobilisierte Enzyme**

(71) Anmelder: Evonik Degussa GmbH, 45128 Essen (DE)
(72) Erfinder: Eckstein, Marrit Friederike, 45289 Essen (DE); Drexel, Claus-Peter, 63263 Neu-Isenberg (DE)

(57) **Zusammenfassung**

Oberflächenmodifizierter, poröser Träger oder oberflächenmodifiziertes, enzymhaltiges Korn, deren Oberfläche mit Metalloxidpartikeln belegt ist, wobei die Metalloxidpartikel aus der Gruppe bestehend aus Aluminiumoxid, Calciumoxid, Magnesiumoxid, Manganoxid, Titandioxid und/oder Mischoxiden dieser Metalloxide ausgewählt sind.

## Beschreibung

Die Erfindung betrifft mit Metalloxidpartikeln oberflächenmodifizierte Träger und immobilisierte Enzyme, sowie Verfahren zu deren Herstellung.

In WO97/039116 wird ein Verfahren zur Herstellung eines enzymhaltigen Partikels offenbart. Zur Verringerung der statischen Aufladung wird empfohlen ein Gleitmittel, vorzugsweise Polyethylenglykole (PEGs) oder ethoxylierte Fettalkohole, einzusetzen. Weiterhin ist eine Beschichtung aus Polyethylenglykolen, Fettalkoholen, ethoxylierten Fettalkoholen, Fettsäuren, Mono-, Di- oder Triglyceriden von Fettsäuren, Polyolen, Zuckern, Zuckeralkoholen, Harnstoff oder Phthalatestern vorgesehen.

Wiemann berichtet in seiner Dissertationsschrift vom 28. September 2010, Technische Universität Berlin, über Verfahren zur Herstellung stabiler, siliconbeschichteter Enzympräparate. Darin werden die Probleme der Beschichtung des kommerziell erhältlichen Lipaseimmobilisates Novozym^{®} 435, Fa. Novozymes, mit Siliconmonomeren mittels eines Wirbelschicht-Verfahrens geschildert. Die dem Fachmann bekannte Verfahrensweise die Prozessluft anzufeuchten führt hier zu einer unerwünschten Agglomerierung der Immobilisate.

Die Aufgabe der vorliegenden Erfindung bestand daher in der Bereitstellung eines porösen Trägers oder eines enzymhaltiges Korns, die bei einer Prozessierung keine Agglomerate bilden und weitestgehend homogen und vollständig zu beschichten sind.

Ein erster Gegenstand der Erfindung ist ein oberflächenmodifizierter, poröser Träger oder ein oberflächenmodifiziertes, enzymhaltiges Korn, deren Oberfläche mit Metalloxidpartikeln belegt ist, wobei die Metalloxidpartikel aus der Gruppe bestehend aus Aluminiumoxid, Calciumoxid, Magnesiumoxid, Manganoxid, Titandioxid und/oder Mischoxiden dieser Metalloxide ausgewählt sind.

In einer bevorzugten Ausführungsform weisen mindestens 90% der oberflächenmodifizierten, porösen Träger oder der oberflächenmodifizierten, enzymhaltigen Körner einen Durchmesser von 10 bis 5000 µm, vorzugsweise von 50 µm bis 2000 µm, auf.

Im Rahmen der Erfindung wird unterschieden zwischen einem oberflächenmodifizierten, porösen Träger und einem porösen Träger beziehungsweise einem oberflächenmodifizierten, enzymhaltigen Korn und einem enzymhaltigen Korn. Oberflächenmodifiziert bedeutet dabei, dass die Oberfläche mit Metalloxidpartikeln belegt ist. Fehlt der Term "oberflächenmodifiziert" so handelt es sich um einen porösen Träger oder ein enzymhaltiges Korn, die nicht mit einem der Metalloxidpartikel belegt sind.

Die Metalloxidpartikel im Rahmen der Erfindung weisen bevorzugt eine Partikelgröße von 5 nm bis 100 µm auf. Sie können als isolierte Einzelpartikel und/oder aggregierte Partikel vorliegen. Bei den aggregierten Partikeln handelt es sich um dreidimensionale Sekundärstrukturen von miteinander verwachsenen Primärpartikeln. In einer speziellen Ausführungsform weisen die aggregierten Partikel eine dreidimensionale Struktur auf. Isolierte Einzelpartikel weisen eine annähernd sphärische Gestalt auf und besitzen in der Regel eine Partikelgröße, die in diesem Fall durch einen Partikeldurchmesser von 5 nm bis 100 nm charakterisiert ist. Aggregierte Partikel weisen eine Partikelgröße von 30 nm bis 100 µm, bevorzugt 50 nm bis 50 µm, auf. Bei aggregierten Partikeln bezieht sich die Partikelgröße auf die durch SEM-Aufnahmen bestimmbare größte Ausdehnung eines Aggregates auf dem oberflächenmodifizierten, porösen Träger oder dem oberflächenmodifizierten enzymhaltigen Korn. Aggregate umfassen im Rahmen der Erfindung auch Agglomerate, die durch ein loses Zusammenlagern von Aggregaten gebildet werden.

Die Belegung durch die Metalloxidpartikel muss den porösen Träger oder das enzymhaltige Korn nicht vollständig umgeben. Vielmehr hat es sich gezeigt, dass eine teilweise Umhüllung ausreichend oder sogar vorteilhaft ist. Bevorzugt handelt es sich bei den Metalloxidpartikeln um pyrogen hergestellte Metalloxidpartikel. Diese liegen überwiegend oder ausschließlich in Form von Aggregaten von Primärpartikeln vor. Pyrogen bezieht sich dabei auf die Art ihrer Herstellung mittels einer Flammenhydrolyse oder Flammenoxidation geeigneter Metallverbindungen. Typischerweise tragen die Primärpartikel Hydroxylgruppen auf ihrer Oberfläche und sind frei von Poren. Die BET-Oberfläche der pyrogen hergestellten Metalloxidpartikel liegt im allgemeinen bei 5 bis 300 m²/g. Gute Ergebnisse werden erhalten bei 20 bis 200 m²/g.

Die besten Ergebnisse liefern ein oberflächenmodifizierter, poröser Träger oder ein oberflächenmodifiziertes enzymhaltiges Korn, welche mit pyrogen hergestelltem Aluminiumoxidpartikeln belegt sind. Bevorzugt weist das pyrogen hergestellte Aluminiumoxid eine BET-Oberfläche von 40 bis 150 m²/g. Geeignete kommerziell erhältliche Typen sind beispielsweise AEROXIDE^{®} Alu C, AEROXIDE^{®} Alu 65 oder AEROXIDE^{®} Alu 130, alle von Evonik Industries AG, mit BET-Oberflächen von ca. 100 m²/g, 65 m²/g beziehungsweise 130 m²/g.

Die Wirksamkeit der Metalloxide unterscheidet sich. In der Regel beträgt der Anteil an Metalloxidpartikeln 0,01 bis 5 Gew.-% ist, bezogen auf den oberflächenmodifizierten, porösen Träger oder das oberflächenmodifizierte, enzymhaltige Korn. Die besten Ergebnisse liefern auch hier pyrogen hergestellte Aluminiumoxidpartikel mit einem Anteil von 0,1 bis 2 Gew.-%, bezogen auf den oberflächenmodifizierten, porösen Träger oder das oberflächenmodifizierte enzymhaltige Korn.

Das oberflächenmodifizierte, enzymhaltige Korn im Rahmen der Erfindung umfasst Immobilisate, die Enzyme oder Enzyme enthaltende Mikroorganismen enthalten. Die Immobilisate können ganze Zellen, ruhende Zellen, gereinigte Enzyme oder Zellextrakte, die die entsprechenden Enzyme enthalten oder als Mischungen davon eingesetzt werden. Besonders geeignete Enzyme werden aus der Gruppe bestehend aus Lipasen, Esterasen und Proteasen ausgewählt. Beispielsweise solche aus Candida rugosa, Candida antarctica, Pseudomonas sp., Thermomyces langosiosus, Schweinepankreas, Mucor miehei, Alcaligines sp., Cholesterolesterase aus Candida rugosa, Esterase aus der Schweineleber, besonders bevorzugt Lipasen. Ganz besonders bevorzugt sind Lipasen. Ein weiterer Gegenstand der Erfindung ist ein Verfahren zur Herstellung des oberflächenmodifizierten, porösen Trägers bei dem man einen porösen Träger mit Metalloxidoxidpartikeln in Kontakt bringt. Der poröse Träger weist in der Regel einen mittleren Porendurchmesser von 1 bis 300 nm, besonders bevorzugt 5 bis 100 nm, ganz besonders bevorzugt 10 bis 50 nm, und eine BET-Oberfläche von 1 bis 3000 m²/g, besonders bevorzugt 10 bis 1000 m²/g, ganz besonders bevorzugt 50 bis 300 m²/g, auf.

Als poröse Träger können bevorzugt organische Träger eingesetzt werden. Insbesondere Polyacrylat, Polymethacrylat, Polyvinylstyrol, Styrol-Divinylbenzol-Copolymere, Polypropylen, Polyethylen, Polyethylenterepthalat und/oder PTFE enthalten oder aus diesen bestehen, eingesetzt werden. Als Trägermaterial können saure oder basische Ionenaustauscherharze eingesetzt werden. Beispielsweise Duolite A568, Duolite XAD 761, Duolite XAD 1180, Duolite XAD 7HP, Amberlite IR 120, Amberlite IR 400, Amberlite CG 50, Amberlyst 15, alle Rohm and Haas, oder Lewatit CNP 105, Lewatit VP OC 1600, beide Lanxess^{®}. Besonders bevorzugt sind Polymethacrylate und Polystyrole.

Weitere Gegenstände der Erfindung sind auf Verfahren zur Herstellung des oberflächenmodifizierten, enzymhaltigen Kornes gerichtet. Diese Verfahren sind dadurch gekennzeichnet, dass man
a) ein Enzym mit einem erfindungsgemäßen, oberflächenmodifizierten, porösen Träger in Kontakt bringt,
b) ein auf einem porösen Träger immobilisiertes Enzym mit Metalloxidpartikeln in Kontakt bringt oder
c) Metalloxidpartikel und ein Enzym mit einem porösen Träger in Kontakt bringt.

Ein weiterer Gegenstand der Erfindung ist auf ein Verfahren zum Beschichten eines enzymhaltigen Korns gerichtet, bei dem das erfindungsgemäße oberflächenmodifizierte, enzymhaltige Korn eingesetzt wird. Mit diesem Verfahren kann die Beschichtung problemlos durchgeführt werden, ohne dass es zu Anhaftungen kommt. Als Beschichtungsmittel kommen beispielsweise Polysiloxane in Frage.

Ein kommerziell erhältliches enzymhaltiges Korn ist beispielsweise Novozym 435, Hierbei handelt es sich um einen inerten makroporösen Träger aus Polymethylmethacrylat (PMMA), auf dessen äußere Porenoberfläche CALB bis zu einer Eindringtiefe von ca. 100 µm adsorptiv gebunden ist. Die Enzymkörner haben einen durchschnittlichen Durchmesser von 0,3-0,9 mm und eine BET-Oberfläche von ca. 80 m²/g.

Inkontaktbringen im Rahmen der Erfindung bezieht sich bevorzugt auf Verfahren, die als Verfahrensschritt ein Wirbelschichtverfahren umfassen.

Als Wirbelschicht im Rahmen der Erfindung werden beispielsweise Schüttungen
a) von porösen Trägern und Metalloxidpartikeln
b) enzymhaltigen Körnern und Metalloxidpartikeln
bezeichnet, die von einem aufwärts gerichteten Fluid- bzw. Gasstrom über eine definierte Wegstrecke mitgerissen und so aufgelockert werden.

Im Beschichtungsprozess werden diese mit einer Beschichtungslösung besprüht. Prozessluft bewirkt ein schnelles Trocknen der aufgebrachten Beschichtung und ermöglicht so die Herstellung homogener Schichten.

Die besten Beschichtungsergebnisse werden erhalten, wenn der Behälter mit einem Wurster-Rohr und einer Bottom-Spraydüse ausgestattet ist.

Der oberflächenmodifizierte, poröse Träger gemäß der Erfindung kann als Katalysatorträger verwendet werden. Das oberflächenmodifizierte, enzymhaltige Korn gemäß der Erfindung kann als Biokatalysator verwendet werden.

### Beispiele

Als enzymhaltiges Korn Novozym 435, Lipase CALB immobilisiert auf Polymethylmethacrylat, eingesetzt.

Es wird ein Wirbelschichtreaktor Mini-Glatt der Firma Glatt, Binzen verwendet.

### Beispiel 1: Enzymgranulat im Wirbelschichtreaktor - Vergleich

60 g Novozym 435 werden bei einer Prozesstemperatur von 60°C im Wirbelschichtreaktor mit Druckluft bei 6 bis 7 bar fluidisiert.

Innerhalb von 5 min ist das Wirbelbett vollständig destabilisiert, und das Enzymgranulat haftet an der Reaktorinnenwand.

### Beispiel 2: Enzymgranulat im Wirbelschichtreaktor - erfindungsgemäß

60 g Novozym 435 und 0,5 Gew.-% AEROXIDE® Alu C, Evonik Industries, werden gemischt, in den Wirbelschichtreaktor eingefüllt und bei einer Prozesstemperatur von 60°C mit Druckluft bei 6 bis 7 bar fluidisiert.

Das Wirbelbett kann problemlos aufrechterhalten werden. Nach 180 min wird der Reaktor geöffnet. Es waren nur geringe Anhaftungen zu sehen.

## Patentansprüche

1. Oberflächenmodifizierter, poröser Träger oder
oberflächenmodifiziertes, enzymhaltiges Korn,
**dadurch gekennzeichnet, dass**
deren Oberfläche mit Metalloxidpartikeln belegt ist, wobei die Metalloxidpartikel aus der Gruppe bestehend aus Aluminiumoxid, Calciumoxid, Magnesiumoxid, Manganoxid, Titandioxid und/oder Mischoxiden dieser Metalloxide ausgewählt sind.

2. Oberflächenmodifizierter, poröser Träger oder
oberflächenmodifiziertes, enzymhaltiges Korn
nach Anspruch 1, **dadurch gekennzeichnet, dass**
mindestens 90% des oberflächenmodifizierten, porösen Trägers oder des oberflächenmodifiziertes, enzymhaltigen Korns einen Durchmesser von 10 bis 5000 µm aufweisen.

3. Oberflächenmodifizierter, poröser Träger oder
oberflächenmodifiziertes, enzymhaltiges Korn
nach den Ansprüchen 1 oder 2, **dadurch gekennzeichnet, dass** die Metalloxidpartikel eine Partikelgröße von 5 nm bis 100 µm aufweisen.

4. Oberflächenmodifizierter, poröser Träger oder
oberflächenmodifiziertes, enzymhaltiges Korn
nach den Ansprüchen 1 bis 3, **dadurch gekennzeichnet, dass** es sich um pyrogen hergestellte Metalloxidpartikel handelt.

5. Oberflächenmodifizierter, poröser Träger oder
oberflächenmodifiziertes, enzymhaltiges Korn
nach den Ansprüchen 1 bis 4, **dadurch gekennzeichnet, dass** es sich um pyrogen hergestellte Aluminiumoxidpartikel handelt.

6. Oberflächenmodifizierter, poröser Träger oder
oberflächenmodifiziertes, enzymhaltiges Korn
nach den Ansprüchen 1 bis 5, **dadurch gekennzeichnet, dass** der Anteil an Metalloxidpartikeln 0,01 bis 5 Gew.-% ist, bezogen auf den porösen Träger oder das enzymhaltige Korn,

7. Oberflächenmodifiziertes, enzymhaltiges Korn
nach den Ansprüchen 1 bis 6, **dadurch gekennzeichnet, dass**
das Enzym aus der Gruppe bestehend aus Lipasen, Esterasen und Proteasen ausgewählt ist.

8. Verfahren zur Herstellung eines oberflächenmodifizierten, porösen Trägers gemäß der Ansprüche 1 bis 6,
**dadurch gekennzeichnet, dass**
man einen porösen Träger mit Metalloxidoxidpartikeln in Kontakt bringt.

9. Verfahren zur Herstellung eines oberflächenmodifizierten, enzymhaltigen Kornes gemäß der Ansprüche 1 bis 7,
**dadurch gekennzeichnet, dass**
man ein Enzym mit einem oberflächenmodifizierten, porösen Träger gemäß der Ansprüche 1 bis 5 in Kontakt bringt.

10. Verfahren zur Herstellung eines oberflächenmodifizierten, enzymhaltigen Kornes gemäß der Ansprüche 1 bis 7,
**dadurch gekennzeichnet, dass**
man ein auf einem porösen Träger immobilisiertes Enzym mit Metalloxidpartikeln in Kontakt bringt.

11. Verfahren zur Herstellung eines oberflächenmodifizierten, enzymhaltigen Kornes gemäß der Ansprüche 1 bis 7,
**dadurch gekennzeichnet, dass**
man Metalloxidpartikel und ein Enzym mit einem porösen Träger in Kontakt bringt.

12. Verfahren zum Beschichten eines enzymhaltigen Korns umfassend ein auf einem Träger immobilisiertes Enzym,
**dadurch gekennzeichnet, dass**
das oberflächenmodifizierte, enzymhaltige Korn gemäß der Ansprüche 1 bis 7 eingesetzt wird.

13. Verfahren nach den Ansprüchen 8 bis 12,
**dadurch gekennzeichnet, dass**
das Inkontaktbringen ein Wirbelschichtverfahren umfasst.

14. Verwendung des oberflächenmodifizierten, porösen Trägers gemäß der Ansprüche 1 bis 6 als Katalysatorträger.

15. Verwendung des enzymhaltiges Korns gemäß der Ansprüche 1 bis 7 als Biokatalysator.
